# EUROPEAN PATENT APPLICATION

(11) **EP 4 830 623 A1**
(43) Date of publication of application: **28.10.2026**
(21) Application number: 24906482.5
(22) Date of filing: 19.12.2024
(51) Int. Cl.: A61B 5/145

(54) **MONITORING APPARATUS**

(30) Priority: 19.12.2023 CN 202311756385
(71) Applicant: SHENZHEN SISENSING CO., LTD., Shenzhen Guangdong 518110 (CN)
(72) Inventor: WU, Jiang, Shenzhen, Guangdong 518110 (CN)
(74) Representative: Vossius & Partner Patentanwälte Rechtsanwälte mbB
(86) International application number: PCT/CN2024/140762
(87) International publication number: WO 2025/131007

(57) **Abstract**

The present disclosure describes a monitoring device(1), for application to a body surface of a host(6) to monitor physiological information of the host(6), including a sensor(30), a connector(40), a first housing(10), a fixing member(50), and a second housing(20); the sensor(30) includes a connection portion(32) and an implantable portion(31) implantable under the skin of the host(6), the connection portion(32) has electrical contacts; the sensor(30) is electrically connected to the connector(40) via electrical contacts; the first housing(10) has an accommodation part(16) for accommodating the connector(40) and the connection portion(32); a fixing member(50) is configured to fix the connector(40) and the connection portion(32) in the accommodation part(16); the second housing(20) includes a circuit structure electrically connected to the connector(40). According to the present disclosure, a monitoring device(1) that improves the sealing performance and stability of a sensor(30) is provided.

## Description

### BACKGROUND OF INVENTION

The present disclosure relates to the field of biomedical engineering, and particularly to a monitoring device.

### Description of Related Art

Diabetes mellitus is a common chronic disease, generally caused by insufficient insulin or ineffective insulin action in the body. When blood glucose levels remain elevated over a long period, it may lead to various complications affecting health, making real-time monitoring of blood glucose concentration particularly important. CGM (Continuous Glucose Monitoring) is a continuous glucose monitoring technology that generally includes a sensor assembly and an applicator that is electrically connected to the sensor assembly and attached to the body surface of a user. The sensor assembly may be used to acquire analyte information (e.g., glucose concentration) in the user's body, which is then transmitted by the applicator to the user's smart device or terminal, so that the user can determine his or her blood glucose level in a timely manner. The sensor assembly may generally be divided into a sensor part implanted under the skin of the user (which may be simply referred to as an implantable portion) and a sensor part not implanted under the skin of the user (which may be simply referred to as a connection portion). The connection portion has a plurality of sensor electrical contacts configured to electrically connect to a circuit board on the applicator to transmit electrical signals.

In the prior art, a connector for stabilizing the connection portion and providing a certain sealing effect is generally provided, and the connector can be used to electrically connect the sensor to the circuit board. Typically, the connector and the circuit board are provided on the upper housing of the applicator, and the sensor assembly is provided at a corresponding location on the lower housing of the applicator. When the upper housing is attached to the lower housing, the connector may press the connection portion while electrically connecting the connection portion to the circuit board to transmit an electrical signal.

However, in the above-described technique, when the upper housing and the lower housing are loosened, the connector and the connection portion may separate, and the electrical contacts of the connection portion may be exposed. Product failure may occur when the exposed electrical contacts are affected by external factors, such as exposure to water or moisture. In addition, when the connector is not in secure contact with the connection portion, the connection portion may have difficulty remaining stable, which may cause the sensor to fall off.

### SUMMARY OF INVENTION

The present disclosure has been made in view of the above-mentioned state of the art, and an object of the present disclosure is to provide a monitoring device capable of improving the sealing performance and stability of a sensor.

To this end, the present disclosure provides a monitoring device that is applied to a body surface of a host to monitor physiological information of the host, including a sensor, a connector, a first housing, a fixing member, and a second housing; the sensor including a connection portion and an implantable portion to be implanted subcutaneously in the host, the connection portion having electrical contacts; the sensor being electrically connected to the connector via the electrical contacts; the first housing having an accommodation part for accommodating the connector and the connection portion; the fixing member being configured to fix the connector and the connection portion in the accommodation part; and the second housing including a circuit structure electrically connected to the connector.

In the monitoring device according to the present disclosure, the connection portion of the sensor is electrically connected to the circuit structure of the second housing via the connector, and the fixing member fixes the connector and the connection portion in the accommodation part of the first housing. In this case, the connector can prevent the electrical contacts of the connection portion from being exposed (i.e., keeping the connection portion in a sealed state), and can reduce negative effects (e.g., malfunction or contamination of the connection portion) when the electrical contacts of the connection portion come into contact with the external environment when the first housing and the second housing are loosened or separated. In addition, the fixing member can stably hold the connector and the connection portion within the accommodation part, reduce looseness of the connector and the connection portion, and make the connection between the connector and the connection portion more secure, thereby further reducing the exposure of the electrical contacts and improving the sealing performance of the connection portion of the sensor.

In addition, in the monitoring device according to the present disclosure, optionally, the connector has conductors aligned with and equal in number to the electrical contacts, one end of the conductors being connected to the electrical contacts, and the other end of the conductors being connected to the circuit structure. In this case, since the sensor generally transmits an electrical signal via the electrical contacts, by providing conductors that are aligned with and equal in number of the electrical contacts, the electrical signal of each electrical contact of the sensor can be transmitted to the circuit structure via its corresponding conductor, thereby reducing current instability and improving the reliability and stability of the electrical connection between the sensor and the circuit structure.

In addition, in the monitoring device according to the present disclosure, optionally, the connector includes an inner cavity and at least one pivot part for controlling the opening or closing of the connector, the connector being configured to receive the conductor into the inner cavity when in an open state. In this case, the connector can be controlled to open to facilitate manipulation of the conductor (e.g., placement or replacement of the conductor), and at the same time, the connector can be controlled to close, thereby stabilizing the conductor after it is inserted into the inner cavity of the connector.

In addition, in the monitoring device according to the present disclosure, optionally, an upper end of the fixing member has a hollow structure through which the conductor is at least partially exposed so as to electrically connect to the circuit structure. In this case, the conductor within the connector can make more adequate contact with the circuit structure without affecting the fixing member's ability to secure the connector.

In addition, in the monitoring device according to the present disclosure, optionally, the accommodation part has at least one snap protrusion, and a side wall of the fixing member has a snap groove that matches the snap protrusion, the snap groove being configured to snap-fit with the snap protrusion to secure the fixing member and the accommodation part together. In this case, the coupling between the fixing member and the accommodation part can be made tighter, thereby improving the stability of the connection between the connector and the connection portion located within the accommodation part.

In addition, in the monitoring device according to the present disclosure, optionally, the first housing has a first through-hole, and the implantable portion extends outward through the first through-hole. In this case, when the monitoring device or the first housing is applied to the body surface of the host, the implantable portion of the sensor can be inserted subcutaneously in the host.

In addition, in the monitoring device according to the present disclosure, optionally, the connector has at least one pair of sheet structures disposed opposite each other, the sheet structures have a second through-hole; the accommodation part having a column extending through the second through-hole to connect the sheet structures. In this case, by passing the column through the second through-hole to connect the sheet structures, the connector can be fixedly attached to the accommodation part, thereby improving the stability of the connector provided in the accommodation part.

In addition, in the monitoring device according to the present disclosure, optionally, an end surface of the connector where the fixing member is coupled to has a flange part, and the flange part has elasticity. In this case, when the connector is coupled with the fixing member, the fixing member compresses the flange part, causing it to deform, thereby applying greater pressure to the coupling interface and making the connection more secure. In addition, the deformed flange part can fill gaps generated during coupling, thereby further enhancing the tightness of the coupling.

In addition, in the monitoring device according to the present disclosure, optionally, the connection portion and the connector match an inner contour of the accommodation part; the inner contour of the side wall of the fixing member matching an outer contour of the accommodation part. In this case, loosening of the connector and the connection portion within the accommodation part can be reduced, thereby improving their stability. In addition, the fixing member and the accommodation part can be more securely coupled, and assembly errors can be reduced, thereby improving the ease of assembly between the fixing member and the accommodation part.

In addition, in the monitoring device according to the present disclosure, optionally, the second housing is configured to abut the fixing member when coupled to the first housing. In this case, the second housing can exert a force on the fixing member when coupled with the first housing, thereby further pressing the fixing member against the connector, thus improving the stability of the connector and the connection portion. At the same time, since the connection between the connector and the connection portion is more secure, the sealing performance of the connection portion can be improved.

According to the present disclosure, a monitoring device that improves the sealing performance and stability of a sensor is provided.

### BRIEF DESCRIPTION OF THE DRAWINGS

The present disclosure will now be explained in further detail, by way of example only, with reference to the accompanying drawings.
FIG. 1 is a diagram showing an application scenario of a monitoring device according to an example of the present disclosure.
FIG. 2 is a block diagram showing the structure of a monitoring device according to an example of the present disclosure.
FIG. 3A is a schematic diagram showing the external structure of a monitoring device according to an example of the present disclosure. FIG. 3B is an exploded view showing a monitoring device according to an example of the present disclosure.
FIG. 4A is a plan view showing a first housing according to an example of the present disclosure. FIG. 4B is a schematic diagram showing the assembly of a fixing member and a connector according to an example of the present disclosure.
FIG. 5A is a schematic diagram showing the structure of a connector according to an example of the present disclosure. FIG. 5B is a schematic diagram showing a connector according to an example of the present disclosure in an open state.
FIG. 6 is a schematic diagram showing the structure of a fixing member according to an example of the present disclosure.

Description of reference numerals: 1... monitoring device, 10... first housing, 20... second housing, 30... sensor, 40... connector, 50... fixing member, 6... host, 11... base plate, 12... first through-hole, 13... first side wall, 14... engagement groove, 15... pressing part, 150... first end, 151... second end, 16... accommodation part, 160... column, 161... snap protrusion, 21... second side wall, 22... first edge protrusion, 23... second edge protrusion, 31... implantable portion, 32... connection portion, 41... conductor, 42... inner cavity, 43... connection opening, 44... connection upper part, 45... connection lower part, 46... pivot part, 47... sheet structures, 470... first sheet, 471... second sheet, 472... second through-hole, 48... flange part, 51... hollow structure, 52... snap groove, 53... first block, 54... second block, 55... notch part.

### DETAILED DESCRIPTION

Hereinafter, preferred embodiments of the present disclosure will be described in detail with reference to the accompanying drawings. In the following description, the same reference numerals are assigned to the same components, and the repeated description thereof will be omitted. In addition, the drawings are only schematic, and the proportions of the dimensions of the parts relative to each other, the shapes of the parts, etc., may be different from those in practice.

It should be noted that the terms "comprise" and "have", and any variations thereof, for example, a process, method, system, product, or apparatus that comprises or has a list of steps or units are not necessarily limited to those steps or units expressly listed, but may include or have other steps or units not expressly listed or inherent to such process, method, product, or apparatus.

It should be noted that in this document, relative positional and relative directional terms, such as "upper", "upward", "lower", "downward", "vertical direction", "left", "leftward", "left-side", "toward left", "right", "rightward", "right-side", "toward right", "horizontal direction", "front", "forward", "rear", "rearward", "longitudinal direction", and the like, are with reference to a normal operating posture and should not be considered limiting.

The present disclosure relates to a monitoring device, which is a device for monitoring the physiological information of a host. In some examples, the monitoring device may be applied to the body surface of a host to monitor the physiological information of the host.

In some examples, the physiological information may refer to at least one of a heart rate, a respiratory rate, a body temperature, a blood pressure, or a blood glucose concentration of the host. The physiological information may also include other information indicative of the physiological state of the host, and the monitoring device of the present disclosure is not intended to be limiting in this regard.

In some examples, the monitoring device may be wholly or partially placed within the body of the host. Specifically, the monitoring device may be wholly or partially implanted beneath the body surface of the host and react with an analyte within the host, e.g., may react with an analyte in a body fluid of the host and generate analyte information.

In the present disclosure, the analyte for which the monitoring device is directed may be one or more of glucose, acetylcholine, amylase, bilirubin, cholesterol, chorionic gonadotropin, creatine kinase, creatine, creatinine, DNA, fructosamine, glutamine, growth hormone, hormone, ketone body, lactate, oxygen, peroxide, prostate specific antigen, prothrombin, RNA, thyroid stimulating hormone, or troponin.

A monitoring device in accordance with the present disclosure may also be sometimes referred to as a biological monitoring device, a split-body medical device, an analyte information acquisition device, a body surface application device, or the like. It should be noted that each name is intended to mean that the present disclosure relates to a monitoring device that is applied to the body surface of a host to monitor physiological information of the host, and should not be construed as limiting.

Hereinafter, a monitoring device according to the present disclosure will be described in detail with reference to the accompanying drawings.

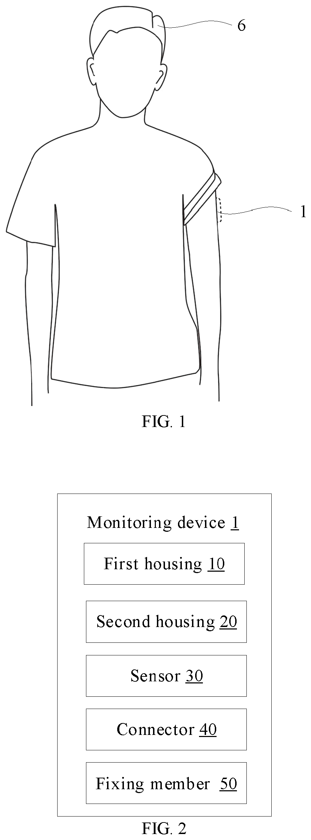

FIG. 1 is a diagram showing an application scenario of a monitoring device 1 according to an example of the present disclosure.

In some examples, referring to FIG. 1, in a scenario, the monitoring device 1 may be applied to a host 6. In some examples, after the sensor of the monitoring device 1 has been implanted wholly or partly beneath the body of the host 6 via an implantable device, the monitoring device 1 may be applied to the body surface of the host 6. In this case, the monitoring device 1 is able to acquire analyte information of the host 6, so that the physiological information of the host 6 can be monitored based on the analyte information.

FIG. 2 is a block diagram showing the structure of a monitoring device 1 according to an example of the present disclosure.

In some examples, referring to FIG. 2, the monitoring device 1 may include a sensor 30, a connector 40, a first housing 10, a fixing member 50, and a second housing 20. The sensor 30 may be configured to acquire analyte information of the host 6. The connector 40 may be electrically connected to the sensor 30 and configured to transmit an electrical signal indicative of the analyte information of the host 6. The fixing member 50 may be configured to fix the sensor 30 and the connector 40. The first housing 10 may be coupled with the second housing 20 and form an inner cavity, and be configured such that, when coupled, the sensor 30, the connector 40, and the fixing member 50 are accommodated within the inner cavity.

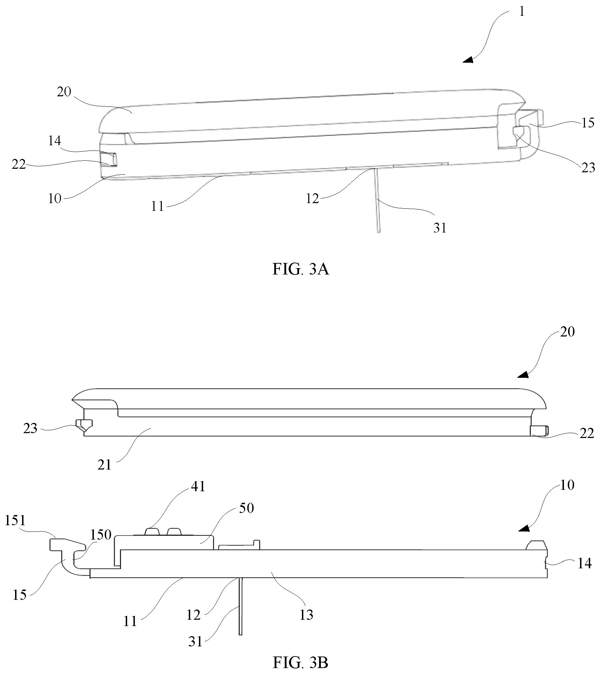

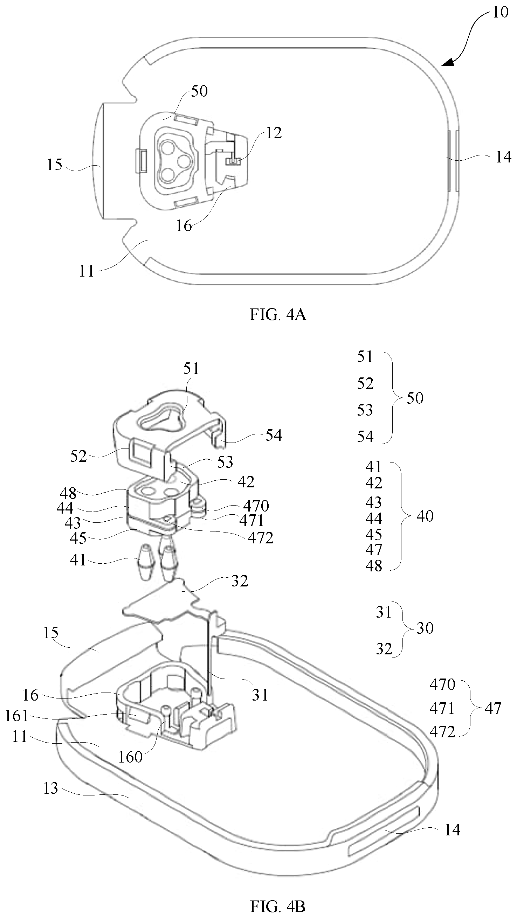

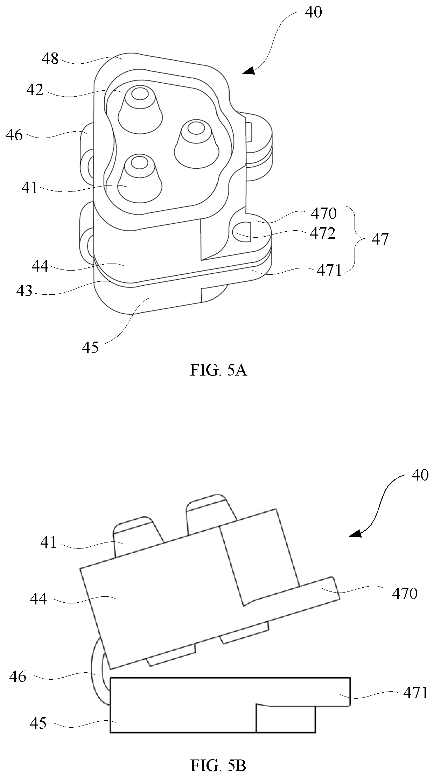

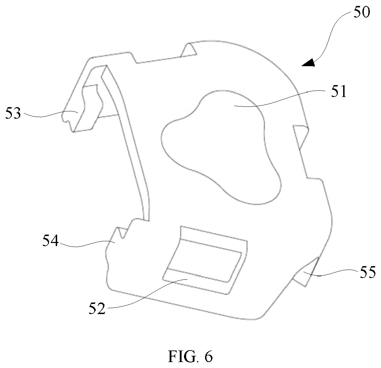

FIG. 3A is a schematic diagram showing the external structure of a monitoring device 1 according to an example of the present disclosure. FIG. 3B is an exploded view showing the monitoring device 1 according to an example of the present disclosure. FIG. 4A is a plan view showing the first housing 10 according to an example of the present disclosure. FIG. 4B is a schematic diagram showing the assembly of the fixing member 50 and the connector 40 according to an example of the present disclosure. FIG. 5A is a schematic diagram showing the structure of a connector 40 according to an example of the present disclosure. FIG. 5B is a schematic diagram showing the connector 40 according to an example of the present disclosure in an open state. FIG. 6 is a schematic diagram showing the structure of a fixing member 50 according to an example of the present disclosure.

In some examples, referring to FIGs. 3A and 3B, the monitoring device 1 may include a first housing 10. In some examples, referring to FIGs. 3A and 3B, the monitoring device 1 may include a second housing 20. In some examples, the first housing 10 may be detachably assembled with the second housing 20.

In some examples, the first housing 10 may have a base plate 11 that may be applied to the body surface of the host 6. In some examples, the first housing 10 may be used to house the sensor 30. Specifically, in some examples, the base plate 11 may be used to house the sensor 30. In some examples, the sensor 30 may include an implantable portion 31 that may be placed subcutaneously in the host 6.

In some examples, the first housing 10 may have a first through-hole 12. In some examples, the first through-hole 12 may be provided on the base plate 11 of the first housing 10. In some examples, the location of the first through-hole 12 in the base plate 11 may match the location of the implantable portion 31 of the sensor 30. In some examples, the location of the first through-hole 12 in the base plate 11 may overlap with the location of the implantable portion 31 of the sensor 30.

In some examples, the implantable portion 31 may extend outwardly along the first through-hole 12. In this case, when the monitoring device 1 or the first housing 10 is applied to the body surface of the host 6, the implantable portion 31 of the sensor 30 can be placed under the skin of the host 6.

In some examples, the implantable portion 31 may include a sensing layer that may be configured to react with an analyte to obtain analyte information.

In some examples, referring to FIG. 3B, the first housing 10 may include a first side wall 13. The first side wall 13 may be connected to the base plate 11. In some examples, the first side wall 13 may be configured to extend in a direction away from the host 6.

In some examples, the first housing 10 may include an engagement groove 14 (see FIGs. 3A and 3B), the engagement groove 14 may be provided in the first side wall 13. In some examples, the engagement groove 14 may be a square hole, a round hole, or an oval hole.

In some examples, the first housing 10 may include a pressing part 15 (see FIGs. 3A and 3B), the pressing part 15 may be provided at the first side wall 13. In some examples, the pressing part 15 may be configured to couple or separate from the first housing 10 and the second housing 20. Specifically, the pressing part 15 may be configured such that the first housing 10 may be coupled to or separated from the second housing 20 when a force is applied to the pressing part 15.

In some examples, the second housing 20 may include a second side wall 21 (see FIG. 3B) that is adapted to the first side wall 13. Specifically, the first side wall 13 may be fully or partially coupled with the second side wall 21 to combine the first housing 10 with the second housing 20.

In some examples, referring to FIG. 3B, the second housing 20 may include a first edge protrusion 22 and a second edge protrusion 23. The first edge protrusion 22 and the second edge protrusion 23 may be provided on the second side wall 21.

In some examples, the first edge protrusion 22 may be configured to engage the engagement groove 14. In some examples, the first edge protrusion 22 may be coupled with the engagement groove 14 by being inserted into the engagement groove 14. Thus, the first housing 10 and the second housing 20 can be coupled to each other.

In some examples, the second edge protrusion 23 may be configured to be inter-coupled with the pressing part 15. In some examples, the pressing part 15 may include a first end 150 configured to engage with the second edge protrusion 23 (see FIG. 3B). The end surface of the first end 150 may conform to the shape of the second edge protrusion 23.

In some examples, the pressing part 15 may include a second end 151, the second end 151 may be configured to control the coupling state between the first end 150 and the second edge protrusion 23. In some examples, the coupling state may include a first state indicating that the second edge protrusion 23 is engaged with the first end 150 and a second state indicating that the second edge protrusion 23 is disengaged from the first end 150.

In some examples, the coupling state may be changed by applying a force to the second end 151 of the pressing part 15; that is, the first state and the second state may be switched by applying a force to the second end 151 of the pressing part 15.

In some examples, the second housing 20 may include a circuit structure. In some examples, the second housing 20 may include a circuit structure that is electrically connected to the sensor 30. In some examples, the circuit structure may include an electronic module that may be configured to process an electrical signal from the sensor 30 for characterizing analyte information. In some examples, the electronic module may be configured to transmit the analyte information to a smart terminal of the host 6. In this case, the host 6 can easily acquire its own analyte information, thereby enabling real-time monitoring of its physiological parameters.

In some examples, the circuit structure may include a power supply module. The power supply module may be configured to provide electrical power to the monitoring device 1 for proper operation of the monitoring device 1. In some examples, the power supply module may be a rechargeable battery.

In some examples, the second housing 20 may be configured to abut the fixing member 50 when coupled with the first housing 10. In this case, the second housing 20 can exert an action on the fixing member 50 when coupled with the first housing 10, thereby further pressing the fixing member 50 against the connector 40, thus improving the stability of the connector 40 and the connection portion 32. Meanwhile, since the connection between the connector 40 and the connection portion 32 is more secure, the sealing performance of the connection portion 32 can be improved.

In some examples, referring to FIGs. 4A and 4B, the first housing 10 may have an accommodation part 16. The accommodation part 16 may be configured to accommodate the connector 40 and the connection portion 32.

In some examples, referring to FIG. 4B, the sensor 30 may include a connection portion 32. The connection portion 32 may be connected to the implantable portion 31.

In some examples, the connection portion 32 may have electrical contacts. In some examples, the connection portion 32 may have at least one electrical contact. For example, the connection portion 32 may have one, two, or three electrical contacts.

In some examples, the sensor 30 may be electrically connected to the connector 40 via the electrical contacts. In other words, in some examples, the electrical signals acquired by the sensor 30 for characterizing analyte information may be transmitted to the circuit structure through the connector 40.

In some examples, the connection portion 32 may match an inner contour of the accommodation part 16. In some examples, the connector 40 may match the inner contour of the accommodation part 16.

In some examples, the connection portion 32 and the connector 40 may match the inner contour of the accommodation part 16. In some examples, matching may mean that the outer periphery of the connection portion 32 and the connector 40 conforms to the inner contour of the accommodation part 16. In this case, loosening of the connector 40 and the connection portion 32 within the accommodation part 16 can be reduced, thereby improving the stability of the connector 40 and the connection portion 32.

In some examples, the inner contour of the side wall of the fixing member 50 may match the outer contour of the accommodation part 16. In this case, the coupling between the fixing member 50 and the accommodation part 16 can be made tighter, and assembly errors can be reduced, thereby improving the ease of assembling the fixing member 50 with the accommodation part 16.

In some examples, the connector 40 may include at least one conductive channel, and conductive material may have the conductive channel. In other words, the conductive channel may be filled with conductive material.

In some examples, the second housing 20 may include a circuit structure that is electrically connected to the connector 40. Specifically, in some examples, the second housing 20 may include a circuit structure that is electrically connected to the conductive material within the conductive channel of the connector 40. In some examples, one end of the conductive channel may contact the connection portion 32, and the other end of the conductive channel may contact the circuit structure. Thus, the connection portion 32 of the sensor 30 can be electrically connected to the circuit structure in the second housing 20 via the connector 40.

In some examples, the connector 40 may have a conductor 41. In some examples, the conductor 41 may be located within a conductive channel. In some examples, the conductor 41 may have conductive properties. In some examples, the connector 40 may have at least one conductor 41.

In some examples, the connector 40 may have a conductor 41 aligned with the electrical contact. In some examples, the connector 40 may have a number of conductors 41 aligned with and equal to the number of electrical contacts. For example, when the number of electrical contacts of the connection portion 32 is three, the connector 40 may have three conductors 41. In some examples, one end of the conductor 41 may be connected to an electrical contact, and the other end of the conductor 41 may be connected to a circuit structure. Specifically, one end of each conductor 41 may be connected to an aligned electrical contact, and the other end of each conductor 41 may be connected to the circuit structure of the second housing 20. In this case, since the sensor 30 generally transmits electrical signals via the electrical contacts, by providing a number of conductors 41 that are aligned with and equal in number to the electrical contacts, the electrical signals from each electrical contact of the sensor 30 can be transmitted to the circuit structure via its corresponding conductor 41, thereby reducing current instability and improving the reliability and stability of the electrical connection between the sensor 30 and the circuit structure.

In some examples, referring to FIG. 4B, the connector 40 may include an inner cavity 42. In some examples, the conductive channel may be provided in the inner cavity 42. In some examples, the conductor 41 may be provided in the inner cavity 42.

In some examples, the connector 40 may include a connection opening 43 (see FIG. 4B). The connection opening 43 may be configured to make the connector 40 in an open state or a closed state. In some examples, the sensor 30 may be positioned within the inner cavity 42 of the connector 40. In some examples, the connection portion 32 of the sensor 30 may be placed at the connection opening 43 of the connector 40. Specifically, when the connector 40 is in an open state, the connection portion 32 of the sensor 30 can be placed into the inner cavity 42 of the connector 40 through the connection opening 43; when the connector 40 is in the closed state, the connector 40 may fully receive the connection portion 32 within the inner cavity 42.

In some examples, the connector 40 may include a connection upper part 44 and a connection lower part 45 (see FIGs. 5A and 5B). The connection upper part 44 and the connection lower part 45 may be formed based on the connection opening 43.

In some examples, referring to FIG. 5A, the connector 40 may include an inner cavity 42 and at least one pivot part 46. The pivot part 46 may be used to control the opening or closing of the connector 40. That is, the pivot part 46 may be used to control the connector 40 to be in an open state or a closed state. In some examples, the connector 40 may be configured to receive the conductor 41 into the inner cavity 42 in an open state. In this case, the conductor 41 can be easily manipulated (e.g., placed or replaced) by controlling the opening of the connector 40, and at the same time, the conductor 41 can be more stably secured within the inner cavity 42 of the connector 40 by controlling the closing of the connector 40.

In some examples, the pivot parts 46 may be provided on both sides of the connection opening 43. In other words, the pivot part 46 may be connected to the connection upper part 44 and the connection lower part 45, respectively. In this case, the connection upper part 44 and the connection lower part 45 can be controlled to move closer to or farther away from each other along the pivot part 46, thereby enabling control of the connector 40 between the open state and the closed state.

In some examples, the connector 40 may include a plurality of pivot parts 46.

In some examples, a conductive channel may be located at the connection upper part 44.

In some examples, the connector 40 may be configured to receive the conductor 41 to the connection upper part 44 in an open state. In some examples, the connection upper part 44 may include a first side in contact with the connection portion 32 of the sensor 30, and a second side in contact with the circuit structure. In this case, one end of the conductor 41 can be electrically connected to the connection portion 32 of the sensor 30 based on the first side, and at the same time, the other end of the conductor 41 can be electrically connected to the circuit structure based on the second side, whereby the electric signal of the sensor 30 can be transmitted to the circuit structure.

In some examples, the connection lower part 45 may include a third side in contact with the connection portion 32 of the sensor 30. In some examples, when the connector 40 is in a closed state, the first side and the third side may be configured to cover and compress the connection portion 32 of the sensor 30. Thus, the sealing performance between the connector 40 and the connection portion 32 can be improved.

In some examples, the connector 40 may be made of a polymeric material. In some examples, the connector 40 may be made of silicone rubber. In this case, since the silicone rubber has elasticity, it is possible to better wrap the connection portion 32 via elastic deformation, whereby the sealing performance between the connector 40 and the connection portion 32 can be further improved.

In some examples, the connector 40 may include a pair of sheet structures 47 (see FIG. 5A). In some examples, the sheet structures 47 may include a first sheet 470 and a second sheet 471 provided opposite each other. In some examples, the sheet structures 47 may be provided along both sides of the connection opening 43. In other words, the first sheet 470 may be provided at the connection upper part 44, and the second sheet 471 may be provided at the connection lower part 45. In some examples, the first sheet 470 may abut the second sheet 471 when the connector 40 is in the closed state.

In some examples, the connector 40 may have at least one pair of sheet structures 47 provided opposite each other. The sheet structures 47 may have a second through-hole 472. In some examples, the accommodation part 16 may have a column 160 extending through the second through-hole 472 to connect with the sheet structures 47 (see FIG. 4B). In this case, by connecting the column 160 to the sheet structures 47 through the second through-hole 472, the connector 40 can be fixedly connected to the accommodation part 16, thereby improving the stability of the connector 40 provided in the accommodation part 16.

In some examples, the diameter of the second through-hole 472 may match the diameter of the bottom surface of the column 160. In some examples, "matching" may mean that the diameter of the second through-hole 472 is less than or equal to the diameter of the bottom surface of the column 160.

In some examples, the connector 40 may have a flange part 48 (see FIG. 5A). Specifically, the end face of the connector 40 to which the fixing member 50 is coupled may have a flange part 48. In some examples, the flange part 48 may be resilient. In this case, when the connector 40 is coupled with the fixing member 50, the fixing member 50 presses the flange part 48 and causes the flange part 48 to deform, thereby applying greater pressure to the coupled portion, thus making the coupling more secure. In addition, the deformed flange part 48 can fill the gap generated during coupling, thereby further enhancing the tightness of the coupling.

In some examples, the end face of the connector 40 to which the fixing member 50 is coupled may refer to the second side of the connection upper part 44. That is, the flange part 48 may be provided on the second side of the connection upper part 44. In some examples, the second side may extend in a direction toward the circuit structure to form the flange part 48.

In some examples, the fixing member 50 may be configured to secure the connector 40 and the connection portion 32 to the accommodation part 16.

In some examples, referring to FIG. 6, the fixing member 50 may have a hollow structure 51. Specifically, the upper end of the fixing member 50 may have a hollow structure 51. In some examples, the upper end of the fixing member 50 may abut the connector 40. Specifically, the upper end of the fixing member 50 may abut the second face of the connection upper part 44. In some examples, the conductor 41 may be at least partially exposed through the hollow structure 51 to electrically connect with the circuit structure. In this case, the conductor 41 in the connector 40 can be brought into more adequate contact with the circuit structure without affecting the ability of the fixing member 50 to secure the connector 40.

In some examples, the conductive material in the conductive channel may also be electrically connected to the circuit structure through the hollow structure 51.

In some examples, the fixing member 50 may have a snap groove 52. In some examples, the snap groove 52 may be configured to secure the fixing member 50 and the accommodation part 16. In some examples, the fixing member 50 may have at least one snap groove 52.

In some examples, with continued reference to FIG. 4B, the accommodation part 16 may have at least one snap protrusion 161. For example, the accommodation part 16 may have one, two, or three snap protrusions 161. In some examples, referring to FIG. 6, the side wall of the fixing member 50 may have a snap groove 52 matching the snap protrusion 161. In some examples, the snap groove 52 may snap-fit with the snap protrusion 161 to secure the fixing member 50 and the accommodation part 16. In this case, the coupling between the fixing member 50 and the accommodation part 16 can be made tighter, thereby improving the stability of the connection between the connector 40 and the connection portion 32 provided in the accommodation part 16.

In some examples, the snap groove 52 matching the snap protrusion 161 may mean that the number of engagement grooves 52 is the same as the number of snap protrusions 161. For example, when the number of the snap protrusions 161 is three, the number of the snap grooves 52 may be three.

In some examples, referring to FIG. 6, the fixing member 50 may include a first block 53 and a second block 54. In some examples, the first block 53 and the second block 54 may be provided opposite each other on an inner wall of the fixing member 50. In some examples, the first block 53 and the second block 54 may be configured to abut the accommodation part 16. Thus, the stability of the connection between the fixing member 50 and the accommodation part 16 can be further improved.

In some examples, referring to FIG. 6, the fixing member 50 may include a notch part 55.

In some examples, the fixing member 50 may include at least one notch part 55. For example, the fixing member 50 may include one, two, or three notch parts 55. In some examples, the notch part 55 may be located on a side wall of the fixing member 50. In some examples, the notch part 55 may be located at an edge of a side wall of the fixing member 50. That is, the notch part 55 may be located at the junction of each face of the side wall of the fixing member 50. In this case, by providing the notch part 55, the peripheral connection (i.e., the side wall) of the fixing member 50 can be interrupted, allowing the side wall of the fixing member 50 to elastically deform outward during assembly of the fixing member 50, thereby facilitating the installation of the fixing member 50 into the accommodation part 16.

In the monitoring device 1 according to the present disclosure, the connection portion 32 of the sensor 30 is electrically connected to the circuit structure of the second housing 20 via the connector 40, and the fixing member 50 fixes the connector 40 and the connection portion 32 to the accommodation part 16 of the first housing 10. In this case, the connector 40 prevents the electrical contacts of the connection portion 32 from being exposed (i.e., keeps the connection portion 32 sealed), and reduces the negative effects (e.g., connecting portion 32 malfunctions or becomes contaminated) caused by exposure of the electrical contacts of the connection portion 32 to the external environment when the first housing 10 and the second housing 20 become loose or separated. In addition, the fixing member 50 can stably hold the connector 40 and the connection portion 32 in the accommodation part 16, reduce loosening of the connector 40 and the connection portion 32, and make the connection between the connector 40 and the connection portion 32 more secure, thereby further reducing exposure of electrical contacts, and thus improving the sealing performance of the connection portion 32 of the sensor 30.

According to the present disclosure, a monitoring device 1 that improves the sealing performance and stability of the sensor 30 can be provided.

Although the present disclosure has been specifically described with reference to the accompanying drawings and examples, it is understood that the above description does not limit the present disclosure in any form. Those skilled in the art may modify and vary the present disclosure as needed without departing from the essential spirit and scope of the present disclosure, and such modifications and variations are within the scope of the present disclosure.

## Claims

1. A monitoring device, for application to a body surface of a host to monitor physiological information of the host, comprising: a sensor, a connector, a first housing, a fixing member, and a second housing; the sensor comprising a connection portion and an implantable portion implantable subcutaneously in the host, the connection portion having electrical contacts; the sensor being electrically connected to the connector via the electrical contacts; the first housing having an accommodation part for accommodating the connector and the connection portion; the fixing member being configured to fix the connector and the connection portion in the accommodation part; the second housing comprising a circuit structure electrically connected to the connector.

2. The monitoring device according to claim 1, wherein the connector has conductors aligned with and equal in number to the electrical contacts, one end of the conductors is connected to the electrical contacts, and the other end of the conductors is connected to the circuit structure.

3. The monitoring device according to claim 2, wherein the connector comprises an inner cavity and at least one pivot part for controlling opening or closing of the connector, the connector is configured to receive the conductor into the inner cavity in an open state.

4. The monitoring device according to claim 2, wherein an upper end of the fixing member has a hollow structure through which the conductor is at least partially exposed for being electrically connected to the circuit structure.

5. The monitoring device according to claim 1, wherein the accommodation part has at least one snap protrusion, and a side wall of the fixing member has a snap groove matching the snap protrusion, the snap groove snap-fits with the snap protrusion to secure the fixing member and the accommodation part together.

6. The monitoring device according to claim 1, wherein the first housing has a first through-hole, and the implantable portion extends outward along the first through-hole.

7. The monitoring device according to claim 1, wherein the connector has at least one pair of sheet structures provided opposite each other, the sheet structures have a second through-hole; the accommodation part has a column extending through the second through-hole to connect the sheet structures.

8. The monitoring device according to claim 1, wherein an end surface of the connector where the fixing member is coupled has a flange part, the flange part has elasticity.

9. The monitoring device according to claim 1, wherein the connection portion and the connector match an inner contour of the accommodation part; an inner contour of the side wall of the fixing member matches an outer contour of the accommodation part.

10. The monitoring device according to claim 1, wherein the second housing is configured to abut the fixing member when coupled to the first housing.
